(19) Europäisches Patentamt
European Patent Office
Office européen des brevets

(11) Veröffentlichungsnummer: **0 100 952**
**A1**

(12) # EUROPÄISCHE PATENTANMELDUNG

(21) Anmeldenummer: **83107262.4**

(22) Anmeldetag: **25.07.83**

(51) Int. Cl.[3]: **C 07 D 249/08**
C 07 D 233/60, C 07 D 233/56
A 01 N 43/64, A 01 N 43/50

(30) Priorität: **05.08.82 DE 3229273**

(43) Veröffentlichungstag der Anmeldung:
**22.02.84 Patentblatt 84/8**

(84) Benannte Vertragsstaaten:
**AT BE CH DE FR GB IT LI NL SE**


(71) Anmelder: **BAYER AG**
**Zentralbereich Patente, Marken und Lizenzen**
**D-5090 Leverkusen 1, Bayerwerk(DE)**

(72) Erfinder: **Elbe, Hans-Ludwig, Dr.**
**Dasnöckel 59**
**D-5600 Wuppertal 11(DE)**

(72) Erfinder: **Reiser, Wolf, Dr.**
**Kiebitzweg 12 a**
**D-5600 Wuppertal 1(DE)**

(72) Erfinder: **Büchel, Karl Heinz, Prof. Dr.**
**Dabringhausener Strasse 42**
**D-5093 Burscheid(DE)**

(72) Erfinder: **Brandes, Wilhelm, Dr.**
**Eichendorffstrasse 3**
**D-5653 Leichlingen(DE)**

(72) Erfinder: **Frohberger, Paul-Ernst, Dr.**
**Willi-Baumeister-Strasse 5**
**D-5090 Leverkusen 1(DE)**

(72) Erfinder: **Lürssen, Klaus, Dr.**
**August-Kierspel-Strasse 89**
**D-5060 Bergisch-Gladbach 2(DE)**


(54) **Substituierte Azolylallylketone und -carbinole.**


(57) Die Erfindung betrifft neue substituierte Azolylallylketone und -carbinole, ein Verfahren zu ihrer Herstellung sowie ihre Verwendung als Fungizide und Pflanzenwachstumsregulatoren.

Die neuen Verbindungen der Formel

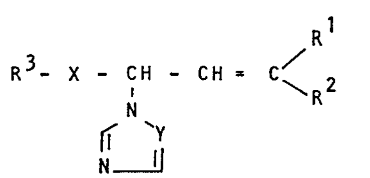

(I)

in welcher $R^1$, $R^2$, $R^3$, X und Y die in der Beschreibung angegebene Bedeutung besitzen, werden erhalten, wenn man Azolylvinylketone in Gegenwart eines Verdünnungsmittels und gegebenenfalls in Gegenwart eines Katalysators bzw. in Gegenwart von Aluminiumoxid erhitzt und gegebenenfalls noch die dabei erhaltenen Azolylallylketone in allgemein üblicher Weise reduziert.

Die Wirkstoffe sind für den Gebrauch als Pflanzenschutzmittel geeignet und können mit besonders gutem Erfolg zur Bekämpfung solcher Pilze eingesetzt werden, die echte Mehltauerkrankungen hervorrufen.


**EP 0 100 952 A1**

Croydon Printing Company Ltd.

BAYER AKTIENGESELLSCHAFT 5090 Leverkusen, Bayerwerk

Zentralbereich
Patente, Marken und Lizenzen Slr-Th
Ia

## Substituierte Azolylallyl-ketone und -carbinole

Die vorliegende Erfindung betrifft neue substituierte Azolylallylketone und -carbinole, ein Verfahren zu ihrer Herstellung sowie ihre Verwendung als Fungizide und Pflanzenwachstumsregulatoren.

Es ist bereits bekannt geworden, daß bestimmte 1-Allyltriazol-Derivate, wie beispielsweise 1-Cyclohexyliden-4,4-dimethyl-2-(1,2,4-triazol-1-yl)-3-pentanon, 1-(Cyclohexen-4-yliden)-4,4-dimethyl-2-(1,2,4-triazol-1-yl)-3-pentanon bzw. -pentanol, 1-Chlor-4-(1,2,4-triazol-1-yl)-2,2,6-trimethyl-5-octen-3-on oder 4-(1,2,4-Triazol-1-yl)-2,2,6-trimethyl-5-hepten-3-ol, gute fungizide und pflanzenwachstumsregulierende Eigenschaften aufweisen (vergleiche DE-OS 29 05 981 [LeA 19 398]). Die Wirkung dieser Verbindungen ist jedoch, insbesondere bei niedrigen Aufwandmengen und -konzentrationen nicht immer voll befriedigend.

Es wurden neue substituierte Azolylallyl-ketone und -carbinole der allgemeinen Formel

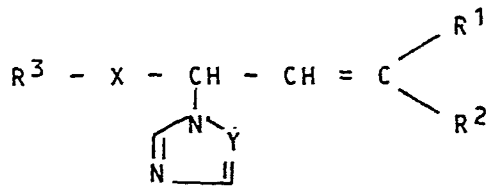

(I)

in welcher

$R^1$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^2$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^1$ und $R^2$ gemeinsam mit dem Kohlenstoffatom, an das sie gebunden sind, für gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl stehen,

$R^3$ für gegebenenfalls substituiertes Cycloalkyl mit 3 bis 5 Kohlenstoffatomen, substituiertes Cyclohexyl oder die Gruppierung

$$R^4-(CH_2)_n-\underset{CH_3}{\overset{CH_3}{\underset{|}{\overset{|}{C}}}}-$$

steht, wobei

$R^4$ für gegebenenfalls substituiertes Phenyl, Alkenyl, Alkinyl, Cyano oder die Gruppierung $-Z-R^5$ steht, wobei

$R^5$ für Alkyl, Halogenalkyl, gegebenenfalls substituiertes Phenyl oder gegebenenfalls substituiertes Phenylalkyl steht und

Z für O, S, SO oder $SO_2$ steht,

n für die Zahlen 0 bis 2 steht,

X für die CO- oder CH(OH)-Gruppe steht und

Y für ein Stickstoffatom oder die CH-Gruppe steht,

sowie deren Säureadditions-Salze und Metallsalz-Komplexe gefunden.

Die erfindungsgemäßen Verbindungen der Formel (I) kommen gegebenenfalls in den geometrischen Isomeren E (trans) und Z (cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Außerdem besitzen die erfindungsgemäßen Verbindungen der Formel(I) für X=CH(OH) zwei asymmetrische Kohlenstoffatome; sie können dann in den beiden geometrischen Isomeren (threo- und erythro-Form) vorliegen, die in unterschiedlichen Mengenverhältnissen anfallen können. In beiden Fällen liegen sie als optische Isomeren vor.

Weiterhin wurde gefunden, daß man die substituierten Azolylallyl-ketone und -carbinole der Formel (I) sowie deren Säureadditions-Salze und Metallsalz-Komplexe erhält, wenn man substituierte Azolylvinyl-ketone der Formel

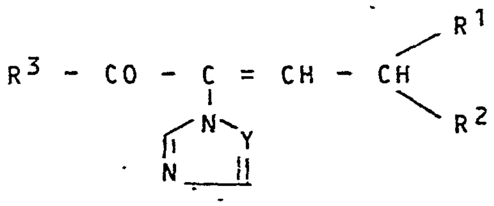 (II) .

in welcher

$R^1$,$R^2$,$R^3$ und Y die oben angegebene Bedeutung haben,

in Gegenwart eines Verdünnungsmittels und gegebenenfalls in Gegenwart eines Katalysators bzw. in Gegenwart von Aluminiumoxid erhitzt und gegebenenfalls die dabei erhaltenen erfindungsgemäßen Azolylallyl-ketone der Formel

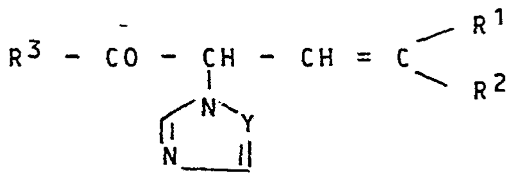 (Ia)

in welcher

$R^1$,$R^2$,$R^3$ und Y die oben angegebene Beduetung haben,

in allgemein üblicher Weise reduziert; und

gegebenenfalls anschließend an die so erhaltenen Verbindungen der Formel (I) eine Säure oder ein Metallsalz addiert.

Schließlich wurde gefunden, daß die neuen substituierten Azolylallyl-ketone und -carbinole der Formel (I) sowie deren Säureadditions-Salze und Metallsalz-Komplexe starke fungizide und starke pflanzenwachstumsregulierende Eigenschaften aufweisen.

Außerdem sind die neuen substituierten Azolylallyl-ketone und -carbinole der Formel (I) interessante Zwischenprodukte zur Herstellung von weiteren Pflanzenschutzmitteln. Bei den Keto-Derivaten kann die Ketogruppe zu einer -CH(OH)-Gruppe bzw. einer -CR(OH)-Gruppe reduziert werden. Ferner können durch entsprechende Umsetzungen funktionelle Derivate der Ketogruppe erhalten werden, wie z.B. Oxime und Oximether, Hydrazone und Ketale. Die Carbinol-Derivate können an der Hydroxy-Gruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der Formel (I) erhalten werden.

Ueberraschenderweise besitzen die erfindungsgemäßen Verbindungen eine bessere fungizide und pflanzenwachstumsregulierende Wirkung als die oben genannten, aus dem Stand der Technik bekannten 1-Allyltriazol-Derivate, welche chemisch und biologisch naheliegende Verbindungen sind. Die erfindungsgemäßen Wirkstoffe stellen somit eine Bereicherung der Technik dar.

Die erfindungsgemäßen substituierten Azolylallyl-ketone und -carbinole sind durch die Formel (I) allgemein definiert. In dieser Formel stehen vorzugsweise

$R^1$ für Wasserstoff; geradkettiges oder verzweigtes Alkyl mit 1 bis 12 Kohlenstoffatomen; geradkettiges oder verzweigtes Alkenyl und Alkinyl mit jeweils 2 bis 7 Kohlenstoffatomen; für gegebenenfalls einfach oder mehrfach, gleich oder verschieden substituiertes Phenyl, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Alkyl und Dialkylamino mit jeweils 1 bis 4 Kohlen-

stoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen, sowie gegebenenfalls durch Halogen und Alkyl mit 1 bis 2 Kohlenstoffatomen substituiertes Phenyl und Phenoxy; weiterhin für gegebenenfalls einfach oder mehrfach, gleich oder verschieden substituiertes Cycloalkyl oder Cycloalkylalkyl mit jeweils 3 bis 7 Kohlenstoffatomen im Cycloalkylteil und 1 bis 2 Kohlenstoffatomen im Alkylteil, wobei als Substituenten vorzugsweise genannt seien: Alykl mit 1 bis 4 Kohlenstoffatomen und Halogen;

$R^2$ für die bei $R^1$ bereits genannten Substituenten;

$R^1$ und $R^2$ gemeinsam mit dem Kohlenstoffatom, an das sie gebunden sind, für jeweils gegebenenfalls einfach oder mehrfach , gleich oder verschieden durch Alykl mit 1 bis 4 Kohlenstoffatomen substituiertes Cycloalykl mit 3 bis 12 Kohlenstoffatomen, Cycloalkenyl mit 3 bis 12 Kohlenstoffatomen, Bicycloalkyl mit 4 bis 24 Kohlenstoffatomen oder Bicycloalkenyl mit 4 bis 24 Kohlenstoffatomen;

$R^3$ für gegebenenfalls einfach oder mehrfach, gleich oder verschieden substituiertes Cycloalkyl mit 3 bis 5 Kohlenstoffatomen, oder substituiertes Cyclohexyl, wobei als Substituenten jeweils infrage kommen; Alkyl mit 1 bis 4 Kohlenstoffatomen und Halogen; sowie für die Gruppierung

$$R^4-(CH_2)_n-\overset{\displaystyle CH_3}{\underset{\displaystyle CH_3}{\overset{|}{\underset{|}{C}}}}- \quad ;$$

$R^4$ für gegebenenfalls einfach oder mehrfach, gleich oder verschieden substituiertes Phenyl, wobei als Substituenten vorzugsweise die bei $R^1$ bereits genannten Phenylsubstituenten infrage kommen; ferner für gerad-

kettiges oder verzweigtes Alkenyl mit 2 bis 4 Kohlenstoffatomen und Alkinyl mit 3 bis 5 Kohlenstoffatomen; für Cyano sowie für die Gruppierung $-Z-R^5$;

$R^5$ für geradkettiges oder verzweigtes Alkyl mit 1 bis 6 Kohlenstoffatomen; Halogenalkyl mit 1 bis 4 Kohlenstoffatomen und 1 bis 5 gleichen oder verschiedenen Halogenatomen, wie Fluor- und Chloratomen; sowie für jeweils gegebenenfalls einfach oder mehrfach, gleich oder verschieden substituiertes Phenyl und Phenylalkyl mit 1 bis 2 Kohlenstoffatomen im Alkylteil, wobei jeweils als Phenylsubstituenten vorzugsweise die bei $R^1$ bereits genannten Phenylsubstituenten infrage kommen;

X,Y,Z und der Index n für die in der Erfindungsdefinition angegebenen Bedeutungen.

Besonders bevorzugt sind diejenigen Verbindungen der Formel (I) in denen

$R^1$ für Wasserstoff, geradkettiges oder verzweigtes Alkyl mit 1 bis 8 Kohlenstoffatomen und geradkettiges oder verzweigtes Alkenyl und Alkinyl mit jeweils 2 bis 4 Kohlenstoffatomen steht; ferner für gegebenenfalls einfach bis dreifach, gleich oder verschieden substituiertes Phenyl steht, wobei als Substituenten genannt seien: Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino und gegebenenfalls durch Fluor, Chlor und Methyl substituiertes Phenyl oder Phenoxy; weiterhin für jeweils gegebenenfalls einfach bis dreifach, gleich oder verschieden durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht;

$R^2$ für die bei $R^1$ bereits genannten Substituenten steht;

$R^1$ und $R^2$ gemeinsam mit dem Kohlenstoffatom, an das sie gebunden sind, für jeweils gegebenenfalls einfach bis dreifach, gleich oder verschieden durch Methyl, Ethyl oder Isopropyl substituiertes Cycloalkyl mit 3 bis 7 Kohlenstoffatomen, Cycloalkenyl mit 3 bis 7 Kohlenstoffatomen, Bicycloalkyl mit 5 bis 12 Kohlenstoffatomen oder Bicycloalkenyl mit 5 bis 12 Kohlenstoffatomen steht;

$R^3$ für gegebenefalls einfach bis dreifach, gleich oder verschieden substituiertes Cyclopropyl oder Cyclopentyl und einfach bis dreifach, gleich oder verschieden substituiertes Cyclohexyl steht, wobei als Substituenten jeweils infrage kommen: Methyl, Ethyl, Isopropyl, tert.-Butyl, Chlor oder Brom; sowie für die Gruppierung

$$R^4 - (CH_2)_n - \overset{\displaystyle CH_3}{\underset{\displaystyle CH_3}{\overset{|}{\underset{|}{C}}}} - \quad \text{steht;}$$

$R^4$ für gegebenenfalls einfach bis dreifach, gleich oder verschieden substituiertes Phenyl steht, wobei als Substituenten vorzugsweise die bei $R^1$ bereits genannten Phenylsubstituenten infrage kommen; ferner für Vinyl, Propargyl, Cyano, sowie die Gruppierung $-Z-R^5$ steht;

$R^5$ für geradkettiges oder verzweigtes Alkyl mit 1 bis 4 Kohlenstoffatomen steht, sowie für jeweils gegebenenfalls einfach bis dreifach, gleich oder verschieden substituiertes Phenyl und Benzyl steht, wobei jeweils als Phenylsubstituenten vorzugsweise die bei $R^1$ bereits genannten Phenylsubstituenten infrage kommen;

X, Y, Z und der Index n für die in der Erfindungsdefinition angegebene Bedeutung stehen.

Bevorzugte erfindungsgemäße Verbindungen sind auch Additionsprodukte aus Säuren und denjenigen Azolylallylketonen und -carbinolen der Formel (I), in denen $R^1$,$R^2$, $R^3$,X und Y die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolyl-allyl-ketonen und -carbinolen der Formel (I), in denen $R^1$, $R^2$, $R^3$, X und Y die Bedeutungen haben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäuren und die Bromwasserstoffsäure, ferner Phosphorsäure, Salpetersäure und Schwefelsäure.

Im einzelnen seien außer den bei den Herstellungsbeispielen genannten Verbindungen folgende Verbindungen der allgemeinen Formel (I) genannt (wobei X für die CO- oder die CH(OH)-Gruppe steht und Y für ein Stickstoffatom oder die CH-Gruppe steht):

Tabelle 1

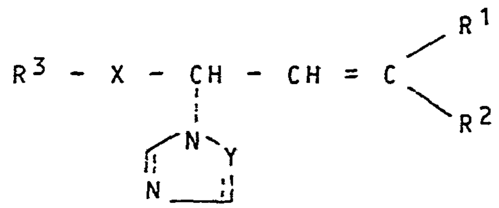

(I)

| $R^3$ | $R^1$ | $R^2$ |
|---|---|---|
| Cl-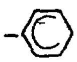-$CH_2$-$C(CH_3)_2$- | H | $CH_3$ |
| Cl-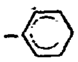-$CH_2$-$C(CH_3)_2$- | H | $C_3H_7$-n |
| Cl-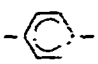-$CH_2$-$C(CH_3)_2$- | H | $C_3H_7$-i |
| Cl-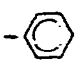-$CH_2$-$C(CH_3)_2$- | H | $C_4H_9$-n |
| Cl-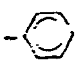-$CH_2$-$C(CH_3)_2$- | H | $C_4H_9$-i |
| Cl-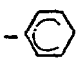-$CH_2$-$C(CH_3)_2$- | $CH_3$ | $CH_3$ |
| Cl-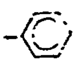-$CH_2$-$C(CH_3)_2$- | $C_2H_5$ | $CH_3$ |
| Cl-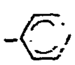-$CH_2$-$C(CH_3)_2$- | $C_2H_5$ | $C_2H_5$ |
| Cl-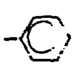-O-$CH_2$-$C(CH_3)_2$- | H | $CH_3$ |
| Cl-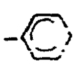-O-$CH_2$-$C(CH_3)_2$- | H | $C_3H_7$-n |
| Cl-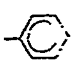-O-$CH_2$-$C(CH_3)_2$- | H | $C_3H_7$-i |
| Cl-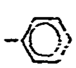-O-$CH_2$-$C(CH_3)_2$- | H | $C_4H_9$-n |
| Cl-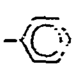-O-$CH_2$-$C(CH_3)_2$- | H | $C_4H_9$-i |
| Cl-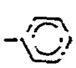-O-$CH_2$-$C(CH_3)_2$- | $CH_3$ | $CH_3$ |
| Cl-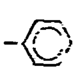-O-$CH_2$-$C(CH_3)_2$- | $C_2H_5$ | $CH_3$ |
| Cl-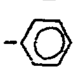-O-$CH_2$-$C(CH_3)_2$- | $C_2H_5$ | $C_2H_5$ |

| $R^3$ | $R^1$ | $R^2$ |
|---|---|---|
| Cl-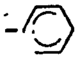-S-$CH_2$-C($CH_3$)$_2$- | H | $CH_3$ |
| Cl-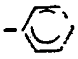-S-$CH_2$-C($CH_3$)$_2$- | H | $C_3H_7$-n |
| Cl-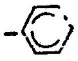-S-$CH_2$-C($CH_3$)$_2$- | H | $C_3H_7$-i |
| Cl-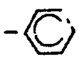-S-$CH_2$-C($CH_3$)$_2$- | H | $C_4H_9$-n |
| Cl-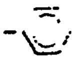-S-$CH_2$-C($CH_3$)$_2$- | H | $C_4H_9$-i |
| Cl-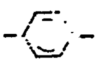-S-$CH_2$-C($CH_3$)$_2$- | $CH_3$ | $CH_3$ |
| Cl-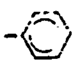-S-$CH_2$-C($CH_3$)$_2$- | $C_2H_5$ | $CH_3$ |
| Cl-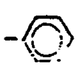-S-$CH_2$-C($CH_3$)$_2$- | $C_2H_5$ | $C_2H_5$ |
| Cl-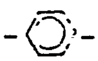-C($CH_3$)$_2$- | H | $CH_3$ |
| Cl-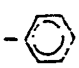-C($CH_3$)$_2$- | H | $C_3H_7$-n |
| Cl-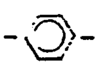-C($CH_3$)$_2$- | H | $C_3H_7$-i |
| Cl-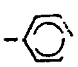-C($CH_3$)$_2$- | H | $C_4H_9$-n |
| Cl-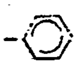-C($CH_3$)$_2$- | H | $C_4H_9$-i |
| Cl-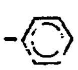-C($CH_3$)$_2$- | $CH_3$ | $CH_3$ |
| Cl-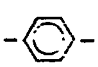-C($CH_3$)$_2$- | $C_2H_5$ | $CH_3$ |
| Cl-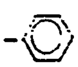-C($CH_3$)$_2$- | $C_2H_5$ | $C_2H_5$ |
| Cl-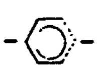-O-C($CH_3$)$_2$- | H | $CH_3$ |
| Cl-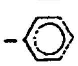-O-C($CH_3$)$_2$- | H | $C_3H_7$-n |
| Cl-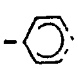-O-C($CH_3$)$_2$- | H | $C_3H_7$-i |
| Cl-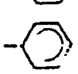-O-C($CH_3$)$_2$- | H | $C_4H_9$-n |
| Cl-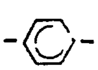-O-C($CH_3$)$_2$- | H | $C_4H_9$-i |
| Cl-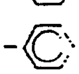-O-C($CH_3$)$_2$- | $CH_3$ | $CH_3$ |
| Cl-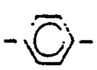-O-C($CH_3$)$_2$- | $C_2H_5$ | $CH_3$ |
| Cl-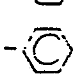-O-C($CH_3$)$_2$- | $C_2H_5$ | $C_2H_5$ |

| $R^3$ | $R^1$ | $R^2$ |
| --- | --- | --- |
| 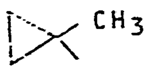 | H | 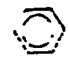 |
| 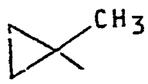 | H | 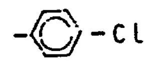 |
| 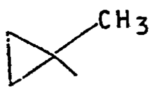 | H | 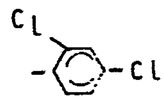 |
| 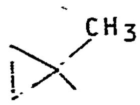 | H | 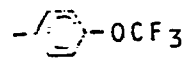 |
| 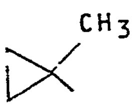 | H | 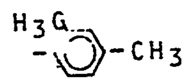 |
| 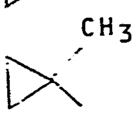 | H | 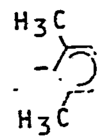 |
| 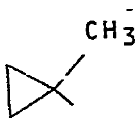 | H | 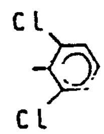 |
| 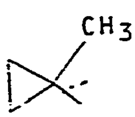 | H | 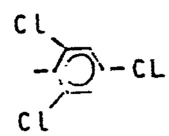 |
| 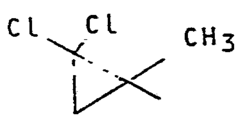 | H | 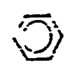 |
| 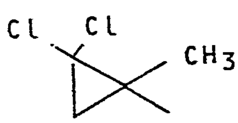 | H | 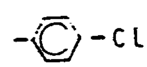 |
| 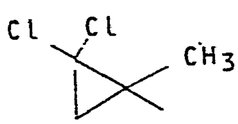 | H | 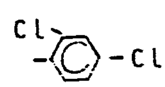 |
| 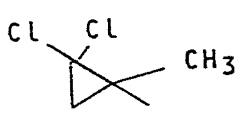 | H | 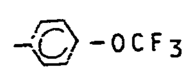 |
| 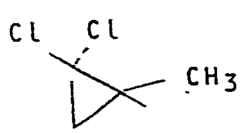 | H | 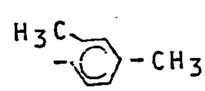 |

| $R^3$ | $R^1$ | $R^2$ |
|---|---|---|
| 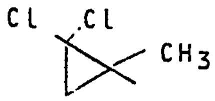 | H | 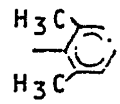 |
| 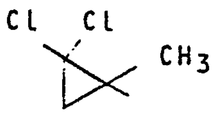 | H | 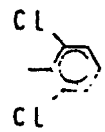 |
| 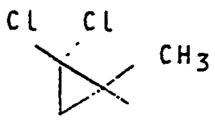 | H | 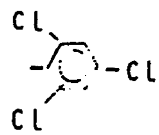 |

Verwendet man beispielsweise 1-(4-Chlorphenyl)-2,2-dimethyl-4-(1,2,4-triazol-1-yl)-4-octan-3-on als Ausgangsstoff, Aluminiumoxid als Reaktionskomponente (Katalysator) und Methanol als Verdünnungsmittel, so kann der Reaktionsablauf nach dem erfindungsgemäßen Verfahren durch das folgende Formelschema wiedergegeben werden:

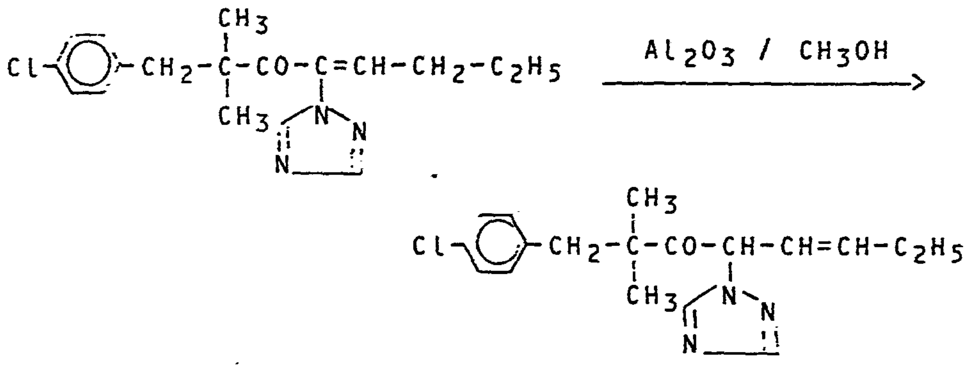


Verwendet man beispielsweise 1-(4-Chlorphenyl)-2,2-dimethyl-4-(1,2,4-triazol-1-yl)-5-octan-3-on als Ausgangsstoff und Natriumborhydrid als Reduktionsmittel, so kann der Reaktionsablauf nach dem erfindungsgemäßen Verfahren durch das folgende Formelschema wiedergegeben werden:

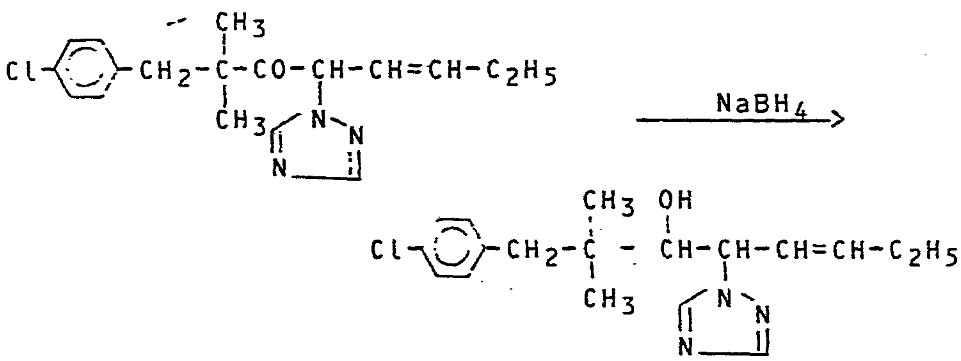


Die bei der Durchführung des erfindungsgemäßen Verfahrens als Ausgangsstoffe benötigten substituierten Azolylvinylketone sind durch die Formel (II) allgemein definiert. In dieser Formel haben $R^1$, $R^2$, $R^3$ und Y vorzugsweise diejenigen Bedeutungen, die bereits im Zusammenhang mit der Beschreibung der erfindungsgemäßen Stoffe der Formel (I)

vorzugsweise für diese Substituenten genannt wurden.

Die substituierten Azolylvinyl-ketone der Formel (II) sind noch nicht bekannt. Sie sind jedoch Gegenstand einer eigenen, parallelen Patentanmeldung und werden erhalten, indem man

b) Ketoenamine der Formel

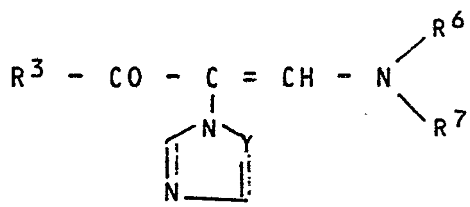

(III)

in welcher

$R^3$ und Y die oben angegebene Bedeutung haben und

$R^6$ und $R^7$ gleich oder verschieden sind und für Alkyl mit 1 bis 4 Kohlenstoffatomen stehen; oder gemeinsam mit dem N-Atom, an das sie gebunden sind, für jeweils gegebenenfalls einfach bis dreifach durch Alkyl mit 1 bis 4 Kohlenstoffatomen substituiertes Piperidinyl, Pyrrolidinyl, Morpholinyl stehen,

mit magnesium-organischen Verbindungen der Formel

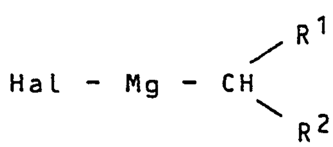

(IV)

in welcher

$R^1$ und $R^2$ die oben angegebene Bedeutung haben und

Hal für Halogen steht,

in Gegenwart eines inerten organischen Lösungsmittels, wie beispielsweise Ether, und gegebenenfalls in Gegenwart eines Inertgases, wie beispielsweise Stickstoff, bei Temperaturen zwischen -20 und 120°C umsetzt (vgl. hierzu auch DE-OS 30 00 643) sowie die Herstellungsbeispiele); oder

b) Azolylketone der Formel

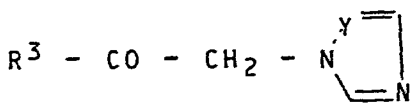 (V)

in welcher

$R^3$ und Y die oben angegebene Bedeutung haben,

mit Aldehyden der Formel

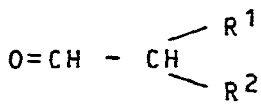 (V I)

in welcher

$R^1$ und $R^2$ die oben angegebene Bedeutung haben,

in üblicher Weise in Gegenwart eines inerten organischen Lösungsmittels, wie beispielsweise Toluol und in Gegenwart eines Katalysators, wie beispielsweise Piperidinacetat, bei Temperaturen zwischen 20 und 160°C umsetzt.

Die Ketoenamine der Formel (III) sind noch nicht bekannt. Sie sind jedoch Gegenstand einer eigenen, parallelen Patentanmeldung und werden erhalten, indem man Azolylketone der Formel

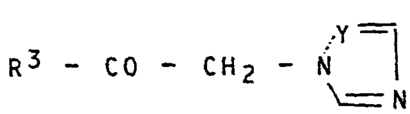 (V)

in welcher

$R^3$ udn Y die oben angegebene Bedeutung haben,

mit Amidacetalen bzw. Aminalestern der Formeln

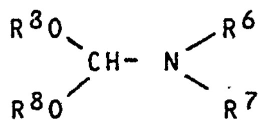

(VIIa)

bzw.

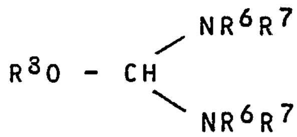

(VII b)

in welcher

$R^6$ und $R^7$ die oben angegebene Bedeutung haben und

$R^8$ für Alkyl mit 1 bis 4 Kohlenstoffatomen steht,

in an sich bekannter Art und Weise in Gegenwart eines inerten organischen Lösungsmittels, wie beispielsweise eines aromatischen Kohlenwasserstoffes und wie insbesondere eines im Ueberschuß eingesetzten Amidacetals bzw. Aminalesters der Formel (VIIa) bzw. (VIIb) in der Siedehitze umsetzt (vergleiche hierzu auch Chem. Ber. 101, 41-50 (1968); J.Org. Chem. 43, 4248-50 (1978) sowie die Herstellungsbeispiele).

Die Azolylketone der Formel (V) sind weitgehend bekannt (vergleiche hierzu DE-OS 24 31 407 /Le A 15 735/, DE-OS 29 06 061 /Le A 19 393/, DE-OS 30 28 330 /Le A 20 458/, DE-OS 30 48 266 /Le A 20 763/, sowie die Deutschen Patentanmeldungen P 31 45 857 vom 19.11.1981 /Le A 21 383/ und P 31 45 858 vom 19.11.1981 /Le A 21 400/; bzw. können sie nach üblichen Methoden hergestellt werden, indem man die entsprechenden

Halogen-ketone in Gegenwart eines Säurebinders mit 1,2,4-Triazol oder Imidazol umsetzt.

Die Amidacetale bzw. Aminalester der Formeln(VII a) bzw. (VII b) sind allgemein bekannte Verbindungen der organischen Chemie (vergleiche z.B. Chem.Ber. 101, 41-50 (1968) und J.Org.Chem. 43, 4248-50 (1978)); bzw. können sie nach den dort angegebenen Verfahren erhalten werden.

Die magnesium-organischen Verbindungen der Formel (IV) sind allgemein bekannte Verbindungen der organischen Chemie, ebenso die Aldehyde der Formel (VI).

Als Verdünnungsmittel kommen für das erfindungsgemäße Verfahren zur Herstellung der Azolylallyl-ketone der Formel (Ia) unter den Reaktionsbedingungen inerte organische Lösungsmittel infrage. Hierzu gehören vorzugsweise Ketone, wie Aceton und Methylethylketon; Alkohole, wie Methanol, Ethanol oder Isopropanol; aliphatische und aromatische Kohlenwasserstoffe, wie Benzol, Toluol oder Xylol; sowie halogenierte Kohlenwasserstoffe, wie Methylenchlorid, Tetrachlorkohlenstoff, Chloroform oder Chlorbenzol.

Das erfindungsgemäße Verfahren zu Herstellung der Azolylallyl-ketone der Formel (Ia) wird gegebenenfalls in Gegenwart einer Base als Katalysator durchgeführt. Hierzu gehören vorzugsweise organische Stickstoffbasen, wie Morpholin, Pyridin, Triethylamin und N,N-Dimethylbenzylamin.

Die Reaktionstemperaturen können bei dem erfindungsgemäßen Verfahren zur Herstellung der Azolylallyl-ketone der Formel (Ia) in eienem größeren Bereich variiiert werden. Im allgemeinen arbeitet man bei Temperaturen zwischen 30 und 150°C, vorzugsweise zwischen 50 und 120°C.

Die Durchführung des erfindungsgemäßen Verfahrens zur Herstellung der Azolyl-allyl-ketone der Formel (Ia) erfolgt entweder rein thermisch durch Erhitzen der Verbindungen der Formel (II), oder in Gegenwart von basischen Katalysatoren, wobei auf 1 Mol der Verbindungen der Formel (II) 0,1 bis 1 Mol an Base eingesetzt wird, oder in Gegenwart von Aluminiumoxid. Die Isolierung der erfindungsgemäßen Verbindungen erfolgt in allen Fällen in üblicher Art und Weise.

Die erfindungsgemäße Reduktion zur Herstellung der Azolyl-allyl-carbinole der Formel (I) erfolgt in üblicher Art und Weise, z.B. durch Umsetzung von Azolyl-allyl-ketonen der Formel (Ia) mit komplexen Hydriden, gegebenenfalls in Gegenwart eines Verdünnungsmittels, oder durch Umsetzung von Azolyl-allyl-ketonen der Formel (Ia) mit Aluminium-isopropylat in Gegenwart eines Verdünnungsmittels.

Arbeitet man mit komplexen Hydriden, so kommen als Verdünnnungsmittel für diese erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ia) etwa 1 Reaktionsäquivalent eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für diese erfindungsgemäße Umsetzung bevorzugt Alkohole, wie Isopropanol, oder inerte Kohlenwasser-

stoffe, wie Benzol, infrage. Die Reaktionstemperaturen können wiederum in einem größeren Bereich veriiert werden; im allgemeinen arbeitet man zwischen 20 und 120°C, vorzugsweise bei 50 bis 100°C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstehende Aluminiumverbindung mit verdünnter Schwefelsäure oder Natronlauge zersetzt. Die weitere Aufarbeitung erfolgt in üblicher Weise.

Zur Herstellung von Säureadditions-Salzen der Azolyl-allyl-ketone und -carbinole der Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen von Azolyl-allyl-ketonen und -carbinolen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalz-Komplexe in bekannter Weise, z.B. durch Abfiltrieren, isolieren und gegebenenfalls durch Umkristallisation reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak,

Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. Getreidemehltaus (Erysiphe graminis), zur Bekämpfung der Blattfleckenkrankheit in Getreide (Pyrenophora teres), zur Bekämpfung der Streifenkrankheit der Gerste (Cochliobolus sativus), zur Bekämpfung von Venturia-Arten, wie z.B. gegen den Erreger des Apfelschorfs (Venturia inaequalis), oder zur Bekämpfung von Reiskrankheiten, wie z.B. Pellicularia sasakii.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,1 und 95 Gewichtsprozent Wirkstoff, vorzugsweise zwischen o,5 und 9o %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50g je kg Saatgut, vorzugsweise 0,01 bis 10 g , benötigt. Bei Behandlung des Bodens sind Wirkstoffkonzentrationen von 0,00001 bis 0,1 Gew.-%, vorzugsweise von 0,0001 bis 0,2 %, am Wirkungsort erforderlich.

Die Herstellung und die Verwendung der erfindungsgemäßen Stoffe werden durch die folgenden Beispiele veranschaulicht.

Herstellungsbeispiele

Beispiel 1

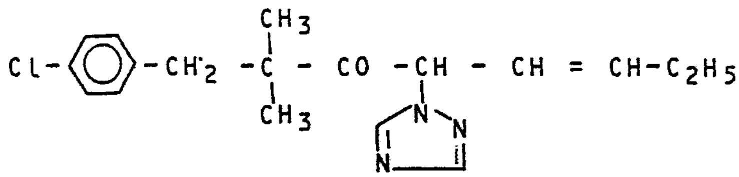

14g (0,042 Mol) 1-(4-Chlorphenyl)-2,2-dimethyl-4-(1,2,4-triazol-1-yl)-4-octen-3-on und 14 g Aluminiumoxid werden in 200 ml Methanol 24 Stunden unter Rückfluß erhitzt. Man läßt das Reaktionsgemisch abkühlen, saugt über Kieselgur ab und engt das Filtrat ein. Man erhält quantitativ 1-(4-Chlorphenyl)-2,2-dimethyl-4-(1,2,4-triazol-1-yl)-5-octen-3-on vom Brechungsindex $n_D^{20}$= 1,5312.

Herstellung des Ausgangsproduktes

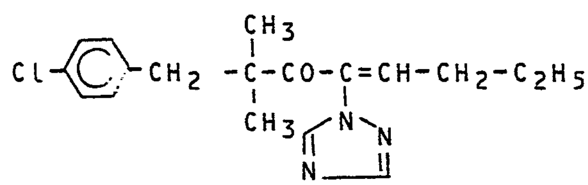

49,9g (0,15 Mol) 1-(4-Chlorphenyl)-2,2-dimethyl-5-dimethylamino-4-(1,2,4-triazol-1-yl)-4-penten-3-on werden in 750 ml Ether gelöst und bei -20°C tropfenweise mit einer Lösung von 33,9g (0,23 Mol) n-Propylmagnesiumbromid in 100 ml Ether versetzt. Man läßt 1,5 Stunden nachrühren, wobei sich das Reaktionsgemisch auf Raumtemperatur erwärmt. Mit verdünnter Salzsäure wird das Reaktionsgemisch auf einen pH-Wert von 7 bis 8 eingestellt. Danach wird die organische Phase abgetrennt, mit Wasser gewaschen, über Natriumsulfat getrocknet und eingeengt. Der Rückstand wird säulenchromatographisch (Kieselgel; Essigester/Cyclohexan= 3:1) gereinigt. Man erhält 30,1g

(60,5 % der Theorie) 1-(4-Chlorphenyl)-2,2-dimethyl-4-(1,2,4-triazol-1-yl)-4-octen-3-on vom Brechungsindex $n_D^{20}$= 1,5429.

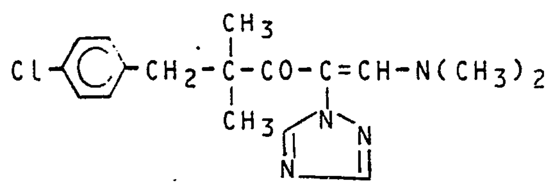

50g (0,18 Mol) 1-(4-Chlorphenyl)-2,2-dimethyl-4-(1,2,4-triazol-1-yl)-3-butanon werden mit 23,6g (0,138 Mol) Dimethylformamid-dimethylacetal 8 Stunden unter Rückfluß erhitzt. Zur Isolierung des Endproduktes wird das Reaktionsgemisch im Vakuum eingeengt. Man erhält 56,5g (94,4 % der Theorie) 1-(4-Chlorphenyl)-2,2-dimethyl-5-dimethylamino-4-(1,2,4-triazol-1-yl)-4-penten-3-on vom Brechungsindex $n_D^{20}$= 1,5797.

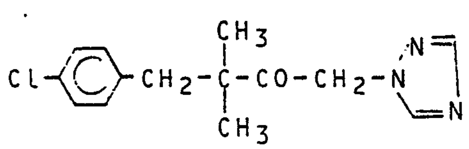

37g (0,13 Mol ) 4-Brom-1-(4-chlorphenyl)-2,2-dimethyl-3-butanon, 13,3g (0,019 Mol) 1,2,4-Triazol und 53,8 g (0,39 Mol) Kaliumcarbonat werden in 300 ml Aceton 8 Stunden unter Rückfluß erhitzt. Man läßt abkühlen, saugt vom anorganischen Rückstand ab und engt das Filtrat ein. Der Rückstand wird in Chloroform aufgenommen, mit Wasser gewaschen, über Natriumsulfat getrocknet und eingeengt. Der Rückstand wird mit Diethylether verrührt, abgesaugt und bei 50°C im Vakuum getrocknet. Man erhält 18,8g (52 % der Theorie) 1-(4-Chlorphenyl)-2,2-dimethyl-4-(1,2,4-triazol-1-yl)-3-butanon vom Schmelzpunkt 127°C.

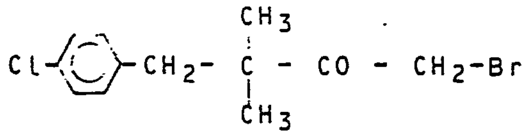

130 g (0,62 Mol) 1-(4-Chlorphenyl)-2,2-dimethyl-3-butanon in 1000 ml Chloroform werden bei Raumtemperatur tropfenweise mit 98,8g (0,62 Mol) Brom versetzt. Man läßt das Reaktionsgemisch 1 Stunde nachrühren und engt anschließend ein. Man erhält 174,7g (97,3 % der Theorie) 4-Brom -1-(4-chlorphenyl)-2,2-dimethyl-3-butanon vom Brechungsindex $n_D^{20}$= 1,5570.

Beispiel 2

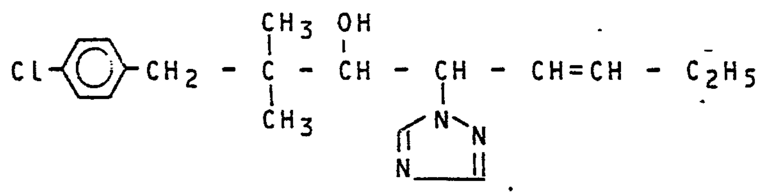

11,3g (0,034 Mol) 1-(4-Chlorphenyl)-2,2-dimethyl-4-(1,2,4-triazol-1-yl)-5-octan-3-on (Beispiel 1 ) werden in 100 ml Methanol gelöst und bei -10°C tropfenweise mit einer Lösung von 0,38 g (0,01 Mol) Natriumborhydrid in 5 ml Eiswasser versetzt. Man läßt 1,5 Stunden bei 0°C nachrühren und stellt dann das Reaktionsgemisch mit verdünnter Salzsäure auf einen pH-Wert von 6 bis 7. Das Reaktionsgemisch wird durch Abdestillieren des Lösungsmittels im Vakuum eingeengt. Der Rückstand wird in Methylenchlorid aufgenommen, mit Wasser gewaschen, über Natriumsulfat getrocknet und eingeengt. Man erhält 10,7g (95 % der Theorie) 1-(4-Chlorphenyl)-2,2-dimethyl-4-(1,2,4-triazol-1-yl)-5-octen-3-ol vom Brechungsindex $n_D^{20}$=1,5340.

In entsprechender Weise und gemäß den angegebenen Verfahren werden die folgenden Verbindungen der Formel (I) erhalten:

Tabelle 2

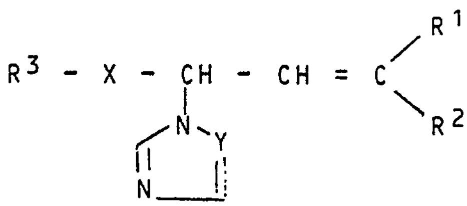

(I)

| Bsp. Nr. | $R^3$ | X | Y | $R^1$ | $R^2$ | Schmelzpunkt(°C) bzw. $n_D^{20}$ |
|---|---|---|---|---|---|---|
| 3 | 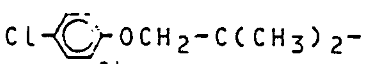 | CO | N | H | $C_2H_5$ | zähes Oel |
| 4 | 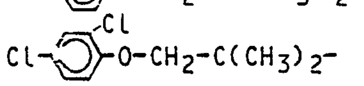 | CH(OH) | CH | H | $C_6H_{13}$ | 1,5352 |
| 5 | 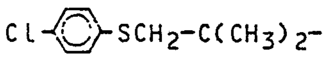 | CO | N | H | $C_2H_5$ | 1,5558 |
| 6 | 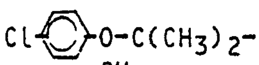 | CO | N | H | $C_2H_5$ | 1,5366 |
| 7 | 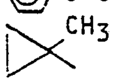 | CO | N | H | $C_4H_9$-i | 1,4938 |
| 8 | 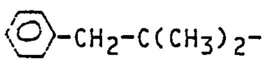 | CO | N | 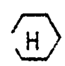 | | zähes Oel |
| 9 | 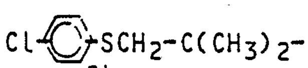 | CO | N | 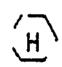 | | zähes Oel |
| 10 | 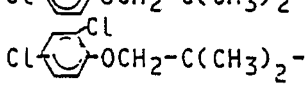 | CO | N | 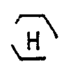 | | zähes Oel |
| 11 | 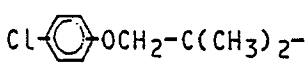 | CH(OH) | N | H | $C_2H_5$ | 30 |
| 12 | 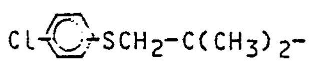 | CH(OH) | N | H | $C_2H_5$ | 1,5631 |
| 13 | 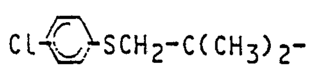 | CH(OH) | N | H | H | 1,5438 |
| 14 | 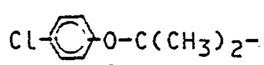 | CH(OH) | N | H | $C_2H_5$ | 1,5300 |
| 15 | 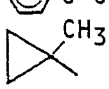 | CH(OH) | N | H | $C_4H_9$-i | 1,4936 |
| 16 | 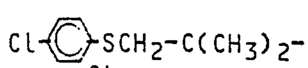 | CH(OH) | H | 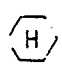 | | zähes Oel |
| 17 | 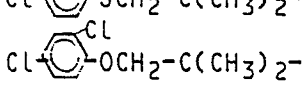 | CO | CH | H | $C_6H_{13}$ | 1,5254 |

Verwendungsbeispiele

In den nachfolgenden Beispielen werden die nachstehend angegebenen Verbindungen als Vergleichssubstanzen eingesetzt:

(A) 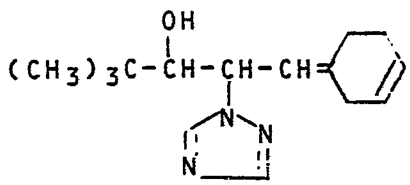

(B) 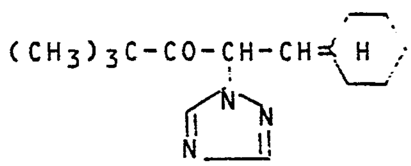

(C) 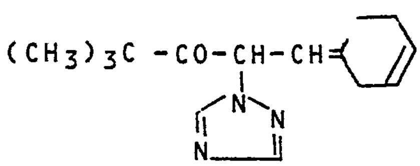

(D) 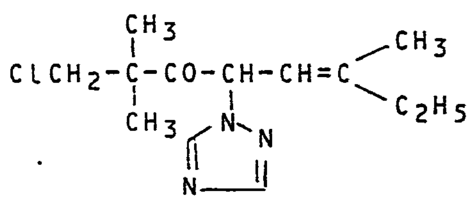

(E) 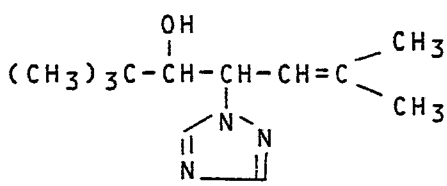

Beispiel A

Venturia-Test (Apfel) / protektiv/

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele:
11.

Beispiel B

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 3,11,6,14,5,1,12,2,1 3,8 und 16.

Beispiel C

Wuchshemmung bei Gerste

Lösungsmittel: 30 Gewichtsteile Dimethylformamid
Emulgator: 1 Gewichtsteil Polyoxyethylen-Sorbitan-monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.
Gerstenpflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Der erfindungsgemäße Wirkstoff 15 zeigt bei diesem Test eine bessere Wuchshemmung als die aus dem Stand der Technik bekannten Verbindungen (D) und (E).

Beispiel D

Wuchsbeeinflussung bei Zuckerrüben

Lösungsmittel: 30 Gewichtsteile Dimethylformamid
Emulgator : 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. in diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 2 und 12 zeigen bei diesem Test eine stärkere Wuchsbeeinflussung als die aus dem Stand der Technik bekannten Verbindungen (A) und (E).

Patentansprüche

1) Substituierte Azolylallyl-ketone und -carbinole der allgemeinen Formel

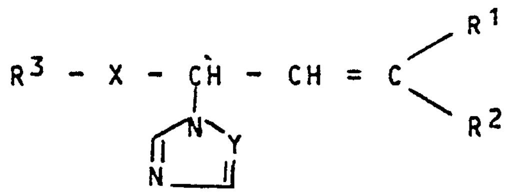

(I)

in welcher

$R^1$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^2$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^1$ und $R^2$ gemeinsam mit dem Kohlenstoffatom, an das sie gebunden sind, für gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl stehen,

$R^3$ für gegebenenfalls substituiertes Cycloalkyl mit 3 bis 5 Kohlenstoffatomen, substituiertes Cyclohexyl oder die Gruppierung

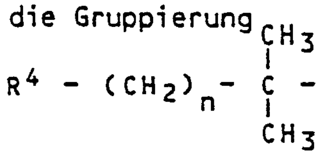

steht, wobei

$R^4$ für gegebenenfalls substituiertes Phenyl, Alkenyl, Alkinyl, Cyano oder die Gruppierung $-Z-R^5$ steht, wobei

$R^5$ für Alkyl, Halogenalkyl, gegebenenfalls substituiertes Phenyl oder gegebenenfalls substituiertes Phenylalkyl steht und

Z für O, S, SO oder $SO_2$ steht,

n für die Zahlen 0 bis 2 steht,

X für die CO- oder CH(OH)-Gruppe steht und

Y für ein Stickstoffatom oder die CH-Gruppe steht,

sowie deren Säureadditions-Salze und Metallsalz-Komplexe.

2) Verbindungen der allgemeinen Formel (I) in Anspruch 1, in welcher

$R^1$ für Wasserstoff, geradkettiges oder verzweigtes Alkyl mit 1 bis 8 Kohlenstoffatomen und geradkettiges oder verzweigtes Alkenyl und Alkinyl mit jeweils 2 bis 4 Kohlenstoffatomen steht; ferner für gegebenenfalls einfach bis dreifach, gleich oder verschieden substituiertes Phenyl steht, wobei als Substituenten genannt seien: Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino und gegebenenfalls durch Fluor, Chlor und

Methyl substituiertes Phenyl oder Phenoxy; weiterhin für jeweils gegebenenfalls einfach bis dreifach, gleich oder verschieden durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht;

$R^2$ für die bei $R^1$ bereits genannten Substituenten steht;

$R^1$ und $R^2$ gemeinsam mit dem Kohlenstoffatom, an das sie gebunden sind, für jeweils gegebenenfalls einfach bis dreifach, gleich oder verschieden durch Methyl, Ethyl oder Isopropyl substituiertes Cycloalkyl mit 3 bis 7 Kohlenstoffatomen, Cycloalkenyl mit 3 bis 7 Kohlenstoffatomen, Bicycloalkyl mit 5 bis 12 Kohlenstoffatomen oder Bicycloalkenyl mit 5 bis 12 Kohlenstoffatomen steht;

$R^3$ für gegebenefalls einfach bis dreifach, gleich oder verschieden substituiertes Cyclopropyl oder Cyclopentyl und einfach bis dreifach, gleich oder verschieden substituiertes Cyclohexyl steht, wobei als Substituenten jeweils infrage kommen: Methyl, Ethyl, Isopropyl, tert.-Butyl, Chlor oder Brom; sowie für die Gruppierung

$$R^4-(CH_2)_n-\underset{CH_3}{\overset{CH_3}{\underset{|}{\overset{|}{C}}}}-$$ steht;

$R^4$ für gegebenenfalls einfach bis dreifach, gleich oder verschieden substituiertes Phenyl steht, wobei als Substituenten vorzugsweise die bei $R^1$ bereits genannten Phenylsubstituenten infrage kommen; ferner für Vinyl, Propargyl, Cyano, sowie die Gruppierung $-Z-R^5$ steht;

$R^5$ für geradkettiges oder verzweigtes Alkyl mit 1 bis 4 Kohlenstoffatomen steht, sowie für jeweils gegebenenfalls einfach bis dreifach, gleich oder verschieden substituiertes Phenyl und Benzyl steht, wobei jeweils als Phenylsubstituenten vorzugsweise die bei $R^1$ bereits genannten Phenylsubstituenten infrage kommen und

X, Y, Z und der Index n die in Anspruch 1 angegebene Bedeutung besitzen.

3) Verfahren zur Herstellung von substituierten Azolylallylketonen und -carbinolen der allgemeinen Formel

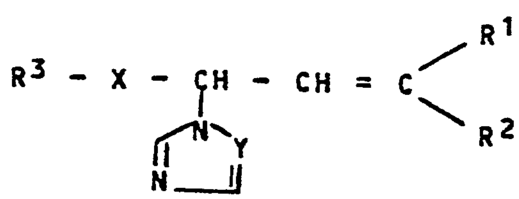

(I)

in welcher

$R^1$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^2$ für Wasserstoff, Alkyl, Alkenyl, Alkinyl, gegebenenfalls substituiertes Phenyl, gegebenenfalls substituiertes Cycloalkyl oder gegebenenfalls substituiertes Cycloalkylalkyl steht,

$R^1$ und $R^2$ gemeinsam mit dem Kohlenstoffatom, an das sie gebunden sind, für gegebenenfalls substituiertes Cycloalkyl, gegebenenfalls substituiertes Cycloalkenyl, gegebenenfalls substituiertes Bicycloalkyl oder gegebenenfalls substituiertes Bicycloalkenyl stehen,

$R^3$ für gegebenenfalls substituiertes Cycloalkyl mit 3 bis 5 Kohlenstoffatomen, substituiertes Cyclohexyl oder die Gruppierung

$$R^4-(CH_2)_n-\overset{\displaystyle CH_3}{\underset{\displaystyle CH_3}{\overset{|}{\underset{|}{C}}}}-$$

steht, wobei

$R^4$ für gegebenenfalls substituiertes Phenyl, Alkenyl, Alkinyl, Cyano oder die Gruppierung $-Z-R^5$ steht, wobei

$R^5$ für Alkyl, Halogenalkyl, gegebenenfalls substituiertes Phenyl oder gegebenenfalls substituiertes Phenylalkyl steht und

Z für O, S, SO oder $SO_2$ steht,

n für die Zahlen 0 bis 2 steht,

X für die CO- oder CH(OH)-Gruppe steht und

Y für ein Stickstoffatom oder die CH-Gruppe steht,

dadurch gekennzeichnet, daß man substituierte Azolyl-vinyl-ketone der Formel

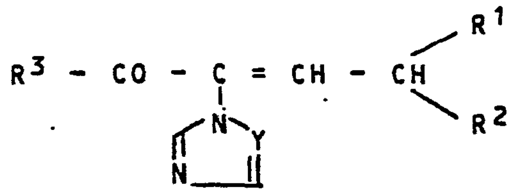 (II)

in welcher

$R^1, R^2, R^3$ und Y die oben angegebene Bedeutung haben,

in Gegenwart eines Verdünnungsmittels und gegebenenfalls in Gegenwart eines Katalysators bzw. in Gegenwart von Aluminiumoxid erhitzt und gegebenenfalls die dabei erhaltenen erfindungsgemäßen Azolylallyl-ketone der Formel

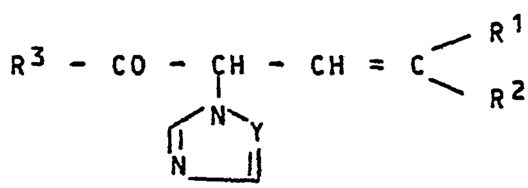 (Ia)

in welcher

$R^1, R^2, R^3$ und Y die oben angegebene Bedeutung haben,

in allgemein üblicher Weise reduziert und

gegebenenfalls anschließend an die so erhaltenen Verbindungen der Formel (I) eine Säure oder ein Metallsalz addiert.

4) Pflanzenschutzmittel, gekennzeichnet durch einen Gehalt an mindestens einem substituierten Azolyl-allyl-keton oder -carbinol der Formel (I) in Ansprüchen 1 und 3.

5) Fungizide und das Wachstum von Pflanzen regulierende Mittel, gekennzeichnet durch einen Gehalt an mindestens einem substituierten Azolylallyl-keton oder -carbinol der Formel (I) in Ansprüchen 1 und 3.

6) Verfahren zur Bekämpfung von Pilzen und zur Regulierung des Pflanzenwachstums, dadurch gekennzeichnet, daß man substituierte Azolyl-ketone oder -carbinole der Formel (I) in Ansprüchen 1 und 3 auf Pilze oder Pflanzen oder ihren Lebensraum einwirken läßt.

7) Verwendung von substituierten Azolylallyl-ketonen oder -carbinolen der Formel (I) in Ansprüchen 1 und 3 als Pflanzenschutzmittel.

8) Verwendung von substituierten Azolylallyl-ketonen oder -carbinolen der Formel (I) in Ansprüchen 1 und 3 zur Bekämpfung von Pilzen und zur Regulierung des Pflanzenwachstums.

9) Verfahren zur Herstellung von Pflanzenschutzmitteln, dadurch gekennzeichnet, daß man substituierte Azolyl-allyl-ketone oder -carbinole der Formel (I) in Ansprüchen 1 und 3 mit Streckmitteln und/oder oberflächenaktiven Mitteln vermischt.

Europäisches Patentamt

# EUROPÄISCHER RECHERCHENBERICHT

0100952
Nummer der Anmeldung

EP 83 10 7262

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 055 833 (BAYER)<br>* Ansprüche; Seiten 70-82 *<br>--- | 1-9 | C 07 D 249/08<br>C 07 D 233/60<br>C 07 D 233/56<br>A 01 N 43/64<br>A 01 N 43/50 |
| Y,D | EP-A-0 054 865 (BAYER)<br>* Ansprüche; Seiten 74-83 *<br>--- | 1-9 | |
| Y,D | EP-A-0 079 006 (BAYER)<br>* Ansprüche; Seiten 40-45 *<br>--- | 1-9 | |
| P,Y | EP-A-0 083 750 (BAYER)<br>* Ansprüche; Seiten 46-49 *<br>--- | 1-9 | |
| Y,D | EP-A-0 016 323 (BAYER)<br>* Ansprüche; Seiten 79-85 *<br>----- | 1-9 | RECHERCHIERTE SACHGEBIETE (Int. Cl. 3)<br>C 07 D 249/00<br>C 07 D 233/00 |

Der vorliegende Recherchenbericht wurde für alle Patentansprüche erstellt.

| Recherchenort | Abschlußdatum der Recherche | Prüfer |
|---|---|---|
| DEN HAAG | 15-11-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82